(19) Europäisches Patentamt
European Patent Office
Office européen des brevets

(11) **EP 2 745 839 B1**

## (12) FASCICULE DE BREVET EUROPEEN

(45) Date de publication et mention
de la délivrance du brevet:
**11.03.2020 Bulletin 2020/11**

(21) Numéro de dépôt: **13198744.8**

(22) Date de dépôt: **20.12.2013**

(51) Int Cl.:
*A61K 31/7048* (2006.01)          *A61P 9/00* (2006.01)
*A61K 9/00* (2006.01)          *A61K 47/36* (2006.01)
*A61K 9/10* (2006.01)

(54) **Composition pharmaceutique sous la forme d'une suspension orale comprenant une fraction flavonoïque et de la gomme xanthane**

Pharmazeutische Zusammensetzung in Form einer Suspension zur Einnahme, die eine flavonoidhaltige Fraktion und Xanthangummi enthält

Pharmaceutical composition in the form of an oral suspension including a flavonoid fraction and xanthan gum

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **21.12.2012 FR 1203579**

(43) Date de publication de la demande:
**25.06.2014 Bulletin 2014/26**

(60) Demande divisionnaire:
**19213282.7**

(73) Titulaire: **Les Laboratoires Servier**
**92284 Suresnes Cedex (FR)**

(72) Inventeurs:
• **Marsas, Stéphanie**
**45430 Checy (FR)**
• **Pean, Jean-Manuel**
**45000 Orleans (FR)**

(56) Documents cités:
**EP-A1- 0 711 560          EP-A1- 0 835 653**
**WO-A1-2009/083759          FR-A1- 2 661 610**

**FR-A1- 2 781 156          FR-M- 6 967**
**US-A1- 2012 070 475**

• **"Recordati OTC Proctolyn Integra Plus", Mintel /GNPD, 1 novembre 2012 (2012-11-01), pages 1-1, XP055058166, Extrait de l'Internet: URL:http://www.gnpd.com/sinatra/gnpd/searc h_results&search_id=rPvvIZ9LtI/&item_id=19 18260 [extrait le 2013-03-28] & "Recordati OTC Proctolyn Integra Plus", , 1 novembre 2012 (2012-11-01), pages 1-1, XP055058167, Extrait de l'Internet: URL:http://www.gnpd.com/sinatra/gnpd/searc h_results&search_id=rPvvIZ9LtI/&item_id=19 18260 [extrait le 2013-03-28]**
• **FRIESENECKER B ET AL: "Oral administration of purified micronized flavonoid fraction suppresses leukocyte adhesion in ischemia-reperfusion injury: In vivo observations in the hamster skin fold", INTERNATIONAL JOURNAL OF MICROCIRCULATION, CLINICAL AND EXPERIMENTAL, BASEL, CH, vol. 14, no. 1-2, 1 janvier 1994 (1994-01-01), pages 50-55, XP009168459, ISSN: 0167-6865**
• **CHAUMEIL J C: "MICRONIZATION: A METHOD OF IMPROVING THE BIOAVAILABILITY OF POORLY SOLUBLE DRUGS", METHODS AND FINDINGS IN EXPERIMENTAL AND CLINICAL PHARMACOLOGY, PROUS SCIENCE, vol. 20, no. 3, 1 avril 1998 (1998-04-01), pages 211-215, XP009048140, ISSN: 0379-0355**

EP 2 745 839 B1

**Description**

La présente invention concerne une suspension orale fortement dosée buvable en sachet comprenant comme principe actif une fraction flavonoïque purifiée et micronisée comprenant de 87% à 93% de diosmine, de 2.5% à 5.0% d'hespéridine, de 0.9% à 2.8% d'isorhoifoline, de 0.9% à 2.8% de linarine et moins de 1% de diosmétine dont la taille des particules est strictement inférieure à 5$\mu$m et comme excipient de la gomme xanthane, ainsi que son utilisation dans le traitement de l'insuffisance veineuse.

La fraction flavonoïque est issue d'un extrait de rutacées. La fraction flavonoïque purifiée et micronisée utilisée dans l'invention contient de 87% à 93% de diosmine et d'autres flavonoïdes de manière concomitante. Ces autres flavonoïdes à hauteur environ de 10% comprennent de 2.5% à 5.0% d'hespéridine, de 0.9% à 2.8% d'isorhoifoline, de 0.9% à 2.8% de linarine et moins de 1% de diosmétine. La taille des particules de la fraction flavonoïque micronisée est strictement inférieure à 5$\mu$m, voire inférieure à 4$\mu$m, 3$\mu$m, 2$\mu$m et même 1.6$\mu$m.

La fraction flavonoïque selon l'invention est administrée à des doses journalières allant de 1000mg à 3000mg afin de traiter les manifestations de l'insuffisance veineuse chronique des membres inférieurs. Du fait d'une importante métabolisation de la fraction flavonoïque dans le tractus gastro-intestinal, ce principe actif doit être fortement dosé à chacune de ses administrations. Par ailleurs, les traitements à base de fraction flavonoïque sont des traitements au long cours imposant une prise facile afin de favoriser l'observance du traitement de la part des patients. En conséquence, il est préférable de développer des formes galéniques permettant une prise facile pour les personnes âgées et sans addition d'eau pour les patients ayant une consommation hors foyer.

L'utilisation en thérapeutique de la fraction flavonoïque extrait de rutacées selon l'invention a été décrite dans le brevet EP 0 711 560. Ce brevet décrit une composition de granulés effervescents fortement dosé à 1000mg de fraction flavonoïque. Cependant ces granulés effervescents sont à disperser dans l'eau avant consommation.

Des solutions buvables contenant des flavonoïdes et de la gomme xanthane ont été décrites dans le brevet US 5,240,732. Ce brevet divulgue une solution dans laquelle les flavonoïdes ont été dissouts dans un alcool contrairement à la présente invention qui concerne une suspension aqueuse dans laquelle la fraction flavonoïque est dispersée de manière homogène.

Des suspensions buvables contenant des polyphénols et des stabilisants de la dispersion ont été décrites dans la demande de brevet US 2012/0070475. Les suspensions selon la demande US 2012/0070475 sont faiblement dosées en quercétine et utilisent exclusivement la gomme gellane en tant que stabilisant de la dispersion.

Enfin, le document FR 6967M divulgue une suspension buvable, devant être agitée avant emploi, de diosmine comprenant 0.5% de gomme adragante.

L'objet de la présente invention est une composition pharmaceutique sous la forme d'une suspension orale fortement dosée buvable en sachet comprenant comme principe actif une fraction flavonoïque purifiée et micronisée comprenant de 87% à 93% de diosmine, de 2.5% à 5.0% d'hespéridine, de 0.9% à 2.8% d'isorhoifoline, de 0.9% à 2.8% de linarine et moins de 1% de diosmétine dont la taille des particules est strictement inférieure à 5$\mu$m et comprenant comme excipient de la gomme xanthane.

Le pourcentage de fraction flavonoïque dans la composition pharmaceutique est compris entre 7%m/m et 20%m/m.

La quantité de fraction flavonoïque dans la composition pharmaceutique est comprise entre 1000mg et 3000mg, dont 2000mg, 1500mg, 2500mg.

La suspension orale fortement dosée en principe actif de la présente invention doit avoir une viscosité adéquate pour permettre d'une part, à la suspension de s'écouler dans les machines de fabrication et hors du sachet lors de l'administration et pour permettre d'autre part, à la fraction flavonoïque de ne pas sédimenter dans le sachet et être stable au cours du temps.

Afin de répondre à la totalité de ces propriétés fonctionnelles, la suspension orale doit répondre à des propriétés rhéologiques spécifiques.

La viscosité de la suspension ne doit pas dépendre du temps pendant lequel elle subit des forces de cisaillement. Il est nécessaire que la suspension retrouve sa viscosité initiale après application d'une contrainte quelques soit sa durée d'application. La viscosité de la suspension doit donc être réversible sur la gamme 0.01 et 1000s$^{-1}$ de cisaillement. Cette propriété rhéologique, i.e. la suspension retrouve entièrement sa viscosité pendant les temps de repos, est essentielle dans les étapes de pompage, préemballage, en passant par le transport et le stockage jusqu'à l'utilisation par le consommateur final de la suspension orale en sachet.

Par ailleurs, il apparait nécessaire que la viscosité de la suspension soit indépendante de la température sur l'intervalle 15°C-60°C, gamme de températures ad hoc à l'issue de la production et au cours des étapes de transport, traitement et emballage du produit final.

Les mesures de viscosité sont réalisées sur le rhéomètre Anton Paar ou sur le rhéomètre de Brookfield. Les résultats obtenus avec ces deux types de rhéomètres sont tout à fait comparables.

Les propriétés rhéologiques et la stabilité requises par la suspension orale selon l'invention sont obtenues à l'aide d'un épaississement de la composition pharmaceutique. L'épaississement de la composition pharmaceutique est obtenu grâce à l'utilisation d'un agent épaississant.

L'agent épaississant selon l'invention est la gomme xanthane. La gomme xanthane est un polysaccharide anionique de haut poids moléculaire constituée de D-glucoses et de D-mannoses comme hexoses dominants. En solution, les molécules de gomme xanthane s'associent entre elles pour former un réseau de molécules enchevêtrées.

La concentration de gomme xanthane dans la composition pharmaceutique selon l'invention est comprise entre 0.45% m/v et 0.55% m/v. La concentration de la gomme xanthane peut être de 0.50% m/v, 0.47%m/v, 0.53%m/v.

La quantité de gomme xanthane dans la composition pharmaceutique est comprise entre 0.30%m/m et 0.60%m/m.

La composition pharmaceutique sous la forme d'une suspension orale fortement chargé en fraction flavonoïque selon l'invention est sans tensioactifs. Les tensioactifs sont généralement utilisés dans les suspensions orales afin d'améliorer la dispersion et la mouillabilité des particules en suspension.

Outre la fraction flavonoïque et la gomme xanthane, la composition pharmaceutique selon l'invention contient un ou plusieurs excipients pharmaceutiquement acceptables tels que des conservateurs, des arômes, des édulcorants ou des correcteurs de pH.

A titre d'exemple d'excipients, on peut citer :

pour les conservateurs : benzoate de sodium, chlorure de benzalkonium, parahydroxybenzoate de méthyle, parahydroxybenzoate de propyle ;
pour les arômes : arôme orange, arôme citron, arôme caramel mou, arôme vanille/citron ;
pour les édulcorants : maltitol, le sorbitol, l'aspartam, le xylitol, l'acésulfame de potassium, la saccharine sodique ;
pour les correcteurs de pH : acide citrique, acide ascorbique, acide tartrique.

La composition pharmaceutique selon l'invention est sous la forme d'une suspension buvable en sachet. Le sachet ou stick a de préférence un volume unitaire de 10ml contenant la dose quotidienne de fraction flavonoïque. Pour une meilleure observance du patient le volume unitaire du sachet ne doit pas être trop important, par exemple 10ml, 12ml, 15ml, 17ml au plus 20ml.

La présente invention concerne également l'utilisation des compositions pharmaceutiques selon l'invention dans le traitement de la maladie veineuse, plus particulièrement de l'insuffisance veineuse. Ces compositions pharmaceutiques sont utilisées comme veinotonique et vasculo-protecteur.

Les exemples ci-dessous illustrent l'invention de manière non limitative.

Exemple 1 : Composition pharmaceutique pour sachet contenant une suspension de 1g de flavonoïdes

| | |
|---|---|
| Benzoate de sodium | 0.015g/10ml |
| Acide citrique | 0.0125g/10ml |
| Arôme orange | 0.015g/10ml |
| Maltitol | 1.8g/10ml |
| Fraction flavonoïque | 1.0g/10ml |
| Gomme xanthane | 0.05g/10ml |
| Eau purifiée | qsp 10ml |

Préparation de la suspension de l'exemple 1 :

Pour environ 100 000 sachets :

700 litres d'eau purifiée et 1.5kg de benzoate de sodium sont soigneusement mélangés à 20°C jusqu'à complète dissolution. 1.25kg d'acide citrique et 1.5kg d'arôme orange sont ajoutés à la solution. 180kg de poudre de maltitol sont ajoutés à la solution et soigneusement mélangés. 100kg de la fraction flavonoïque sont très lentement ajoutés au mélange jusqu'à la formation d'une suspension homogène. 5kg de gomme xanthane est introduite très lentement directement dans la suspension. De l'eau purifiée est ajoutée qsp obtenir un volume final de 1100 litres.

Exemple 2 : Stabilité de la suspension orale de l'exemple 1 (Batch LP02)

La stabilité de la composition pharmaceutique de l'Exemple 1 selon l'invention en sachet a été testée dans différentes conditions de température et d'humidité.

Les sachets sont constitués d'un complexe multicouche (polyéthylène PET12/aluminium AL12 /complexe de polyéthylène extrudé PE50).

| T | Sachet | | | |
|---|---|---|---|---|
| | Densité (Eur. Ph.) | | | |
| | 25°C / 60% HR | 30°C / 65% HR | 30°C / 75% HR | 40°C / 75% HR |
| T0 | 1.11 | | | |
| T0 + 1 mois | 1.10 | 1.10 | 1.10 | 1.10 |
| T0 + 3 mois | 1.10 | 1.10 | 1.10 | 1.10 |

Le tableau ci-dessus relatif à l'évolution de la densité de la suspension dans le sachet montre que la densité est totalement stable même dans des conditions de température et d'humidité élevées (40°C/75%HR). Cette stabilité de la densité montre que la suspension ne déphase pas au cours du temps dans des conditions de température et d'humidité élevées.

| T | Sachet | | | | | | | |
|---|---|---|---|---|---|---|---|---|
| | Taille des particules de la fraction flavonoïque | | | | | | | |
| | 25°C / 60% HR | | 30°C / 65% HR | | 30°C / 75% HR | | 40°C / 75% HR | |
| T0 | | | | | d10 | 0.658 | | |
| | | | | | d50 | 2.581 | | |
| | | | | | d90 | 6.642 | | |
| T0 + 1 mois | d10 | 0.723 | d10 | 0.741 | d10 | 0.739 | d10 | 0.760 |
| | d50 | 2.679 | d50 | 2.692 | d50 | 2.694 | d50 | 2.726 |
| | d90 | 6.969 | d90 | 6.675 | d90 | 6.698 | d90 | 6.685 |
| T0 + 3 mois | d10 | 0.726 | d10 | 0.746 | d10 | 0.751 | d10 | 0.798 |
| | d50 | 2.663 | d50 | 2.779 | d50 | 2.708 | d50 | 2.818 |
| | d90 | 6.670 | d90 | 6.862 | d90 | 6.690 | d90 | 6.739 |

La distribution de taille des particules de la fraction flavonoïque est exprimée en diamètre d10, d50 et d90 où d10=d(v,0.1) correspond à la valeur à 10% de la courbe cumulée de la distribution granulométrique en volume; d50=d(v,0.5) correspond à la valeur à 50% de la courbe cumulée de la distribution granulométrique en volume; d90=d(v,0.9) correspond à la valeur à 90% de la courbe cumulée de la distribution granulométrique en volume.

Le tableau ci-dessus montre que la taille des particules de la fraction flavonoïque selon l'invention n'augmente pas au cours du temps dans des conditions de température et d'humidité élevées. Les particules de la fraction flavonoïque micronisée ne s'agglomèrent pas ni ne sédimentent dans la suspension orale selon l'invention.

| T | Sachet | | | |
|---|---|---|---|---|
| | Viscosité en CP | | | |
| | 25°C / 60% HR | 30°C / 65% HR | 30°C / 75% HR | 40°C / 75% HR |
| T0 | 321 | | | |
| T0 + 1 mois | 293 | 298 | 294 | 296 |
| T0 + 3 mois | 293 | 293 | 294 | 298 |

Le tableau ci-dessus relatif à la viscosité de la suspension dans le sachet montre que la viscosité est extrêmement stable au cours du temps dans des conditions de température et d'humidité élevées.

Enfin, un contrôle visuel de la suspension dans les différentes conditions de température et d'humidité après 1 mois et 3 mois d'évaluation montre une absence de grumeaux et de bulles dans les sachets.

Exemple 3 : Uniformité de teneur de la composition pharmaceutique selon l'exemple 1

L'essai est effectué sur 10 sachets. Le contenu de chaque sachet est dosé ; une solution de référence de diosmine est utilisée comme contrôle.

La teneur moyenne $X_m$ est exprimée de la façon suivante :

$$X_m = (\Sigma\, X_i)/10$$

La valeur d'acceptation (AV), exprimée en pourcentage de la valeur théorique, est donnée par la formule suivante :

$$AV = (M\text{-}X_m) + k \times s$$

où :

$X_m$ est la teneur moyenne, exprimée en pourcentage de la valeur théorique; M est la valeur de référence, exprimée en pourcentage de la valeur théorique : M=98.5 si $X_m$ < 98.5; M=$X_m$ si 98.5 $\leq X_m \leq$ 101.5; M=101.5 si $X_m$>101.5; k est la constante d'acceptabilité (k=2.4 pour 10 sachets); s est l'écart type des teneurs $X_i$.

| Paramètres d'uniformité de teneur (diosmine) | Lot LP02 1000 litres (sachet) selon l'Exemple 1 | |
|---|---|---|
| | Début ensachage | Fin ensachage |
| Teneur moyenne | 100.5 | 102.1 |
| Ecart-type | 0.9 | 0.7 |
| Valeur d'acceptation (AV) | 2.2 | 2.3 |

Selon la Pharmacopée Européenne, article 2.9.40, une valeur d'acceptation inférieure à 15 signifie que l'uniformité de teneur est conforme (niveau L1). Le tableau ci-dessus montre donc que la diosmine contenue dans la formulation selon l'invention a une uniformité de teneur qui répond aux exigences réglementaires.

**Revendications**

1. Composition pharmaceutique sous la forme d'une suspension orale, buvable en sachet comprenant comme principe actif une fraction flavonoïque purifiée et micronisée comprenant de 87% à 93% de diosmine, de 2.5% à 5.0% d'hespéridine, de 0.9% à 2.8% d'isorhoifoline, de 0.9% à 2.8% de linarine et moins de 1% de diosmétine dont la taille des particules est strictement inférieure à 5$\mu$m et comme excipient de la gomme xanthane.

2. Composition pharmaceutique selon la revendication 1, dans laquelle la quantité de la fraction flavonoïque est comprise entre 7%m/m et 20%m/m.

3. Composition pharmaceutique selon la revendication 1, dans laquelle la concentration de gomme xanthane est de 0.5% m/v.

4. Composition pharmaceutique selon la revendication 1, dans laquelle la quantité de gomme xanthane est comprise entre 0.30%m/m et 0.60%m/m.

5. Composition pharmaceutique selon la revendication 1, comprenant un ou plusieurs excipients pharmaceutiquement acceptables choisis parmi les édulcorants, les arômes, les conservateurs ou les correcteurs de pH.

6. Composition pharmaceutique selon l'une quelconque des revendications 1 à 5, pour son utilisation dans le traitement de l'insuffisance veineuse.

**Patentansprüche**

1. Pharmazeutische Zubereitung in Form einer oralen, trinkbaren Suspension im Sachet, umfassend als Wirkstoff eine gereinigte und mikronisierte Flavonoidfraktion umfassend 87 % bis 93 % Diosmin, 2,5 % bis 5,0 % Hesperidin, 0,9 % bis 2,8 % Isorhoifolin, 0,9 % bis 2,8 % Linarin und weniger als 1 % Diosmetin, wobei die Größe der Teilchen strikt unterhalb 5 $\mu$m liegt, und als Trägermaterial Xanthangummi.

2. Pharmazeutische Zubereitung nach Anspruch 1, worin die Menge der Flavonoidfraktion zwischen 7 % M/M und 20 % M/M liegt.

3. Pharmazeutische Zubereitung nach Anspruch 1, worin die Konzentration des Xanthangummis 0,5 % M/V beträgt.

4. Pharmazeutische Zubereitung nach Anspruch 1, worin die Menge des Xanthangummis zwischen 0,30 % M/M und 0,60 % M/M liegt.

5. Pharmazeutische Zubereitung nach Anspruch 1, umfassend ein oder mehrere pharmazeutisch annehmbare Hilfsstoffe ausgewählt aus Süßungsmitteln, Aromastoffen, Konservierungsmitteln oder pH-Korrektoren.

6. Pharmazeutische Zubereitung nach einem der Ansprüche 1 bis 5 zur Verwendung bei der Behandlung von Veneninsuffizienz.

**Claims**

1. Pharmaceutical composition in the form of a drinkable oral suspension in a sachet, comprising as active ingredient a micronised purified flavonoid fraction comprising from 87% to 93% diosmin, from 2.5% to 5.0% hesperidin, from 0.9% to 2.8% isorhoifolin, from 0.9% to 2.8% linarin and less than 1% diosmetin, the particle size of which is strictly less than 5 $\mu$m, and xanthan gum as excipient.

2. Pharmaceutical composition according to claim 1, wherein the quantity of the flavonoid fraction is between 7% m/m and 20% m/m.

3. Pharmaceutical composition according to claim 1, wherein the concentration of xanthan gum is 0.5% m/v.

4. Pharmaceutical composition according to claim 1, wherein the quantity of xanthan gum is between 0.30% m/m and 0.60% m/m.

5. Pharmaceutical composition according to claim 1, comprising one or more pharmaceutically acceptable excipients chosen from sweeteners, flavourings, preservatives or pH correctors.

6. Pharmaceutical composition according to any one of claims 1 to 5 for use in the treatment of venous insufficiency.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0711560 A
- US 5240732 A
- US 20120070475 A
- FR 6967 M